# FASCICULE DE BREVET EUROPEEN

(11) **EP 3 169 653 B1**
(45) Date de publication et mention de la délivrance du brevet: **29.08.2018**
(21) Numéro de dépôt: 15727015.8
(22) Date de dépôt: 10.06.2015
(51) Int. Cl.: C07C 5/27, C07C 7/13, C07C 15/08

(54) **PROCÉDÉ DE PRODUCTION DE PARAXYLÈNE À HAUTE PURETÉ À PARTIR D'UNE COUPE XYLÈNES, UTILISANT DEUX UNITÉS DE SÉPARATION EN LIT MOBILE SIMULÉ FONCTIONNANT EN SÉRIE ET DEUX UNITÉS D'ISOMÉRISATION**
VERFAHREN ZUR HERSTELLUNG VON HOCHREINEM PARAXYLOL AUS EINER XYLOLFRAKTION MIT ZWEI SIMULIERTEN WANDERBETTTRENNEINHEITEN IN REIHE UND ZWEI ISOMERISIERUNGSEINHEITEN
METHOD FOR THE PRODUCTION OF HIGH-PURITY PARAXYLENE FROM A XYLENE FRACTION, USING TWO SIMULATED MOVING BED SEPARATION UNITS OPERATING IN SERIES AND TWO ISOMERISATION UNITS

(30) Priorité: 18.07.2014 FR 1456940
(43) Date de publication de la demande: 24.05.2017
(73) Titulaire: IFP Energies nouvelles, 92500 Rueil-Malmaison (FR)
(72) Inventeur: DREUX, Héloise, 69007 Lyon (FR); LEFLAIVE, Philibert, 69780 Mions (FR); LEINEKUGEL LE COCQ, Damien, 69600 Oullins (FR)
(86) Numéro de dépôt international: PCT/EP2015/062981
(87) Numéro de publication internationale: WO 2016/008652

(56) Documents cités:
- FR-A1- 2 862 638
- US-A1- 2014 155 667

## Description

### DOMAINE DE L'INVENTION

La production de paraxylène est en constante augmentation depuis trente ans. Les utilisations du paraxylène sont les productions d'acide téréphtalique et de résines polyéthylène téréphtalate, pour fournir des textiles synthétiques, des bouteilles, et plus généralement des matières plastiques.

Pour satisfaire la demande toujours croissante en paraxylène, les pétrochimistes ont le choix entre pratiquer des augmentations de capacité sur des unités existantes (appelé dégoulottage dans la suite du texte), ou construire des unités neuves.

La présente invention permet de répondre à ces deux cas de figure, et plus particulièrement au dégoulottage d'unités existantes, car les modifications impliquées sont relativement modestes.

Dans la suite du texte on parle d'unité de séparation en lit mobile simulé (en abrégé SMB), ou d'unité de séparation (SMB). Une unité de séparation (SMB) peut contenir un ou plusieurs adsorbeurs.

### EXAMEN DE L'ART ANTERIEUR

La production de paraxylène haute pureté par séparation par adsorption est bien connue de l'art antérieur. De manière industrielle, cette opération est réalisée au sein d'un enchainement de procédés dit « boucle C8-aromatique ». Cette « boucle C8-aromatique » inclut une étape d'élimination des composés lourds (c'est-à-dire à plus de 9 atomes de carbone, notés C9+) dans une colonne de distillation appelée « colonne des xylènes ».

Le flux de tête de cette colonne, qui contient les isomères en C8-aromatiques, est ensuite envoyé dans le procédé de séparation du paraxylène qui est généralement un procédé de séparation par adsorption en lit mobile simulé.

L'extrait, qui contient le paraxylène est ensuite distillé au moyen d'une colonne d'extrait puis d'une colonne toluène, pour obtenir du paraxylène de haute pureté.
Le raffinat, riche en métaxylène, orthoxylène et éthylbenzène, après une étape d'élimination du solvant par distillation, est traité dans une unité catalytique d'isomérisation qui redonne un mélange d'aromatiques en C8, dans lequel la proportion des xylènes (ortho-, méta-, paraxylènes) est pratiquement à l'équilibre thermodynamique, et la quantité d'éthylbenzène amoindrie. Ce mélange est à nouveau envoyé dans la « colonne des xylènes » avec la charge fraiche.

L'art antérieur propose de nombreuses variantes de ce schéma de base mettant en oeuvre une ou plusieurs unités de séparation (par adsorption, cristallisation, distillation ou par membrane) et/ou une ou plusieurs unités d'isomérisation en phase gazeuse (convertissant l'éthylbenzène par isomérisation en xylènes ou par désalkylation en benzène), ou en phase liquide (ne convertissant pas l'éthylbenzène).

En particulier, Le brevet FR2862638 décrit un procédé de production de paraxylène à partir d'une charge d'hydrocarbures, dans une colonne d'adsorption travaillant en lit mobile simulé à au moins cinq zones et délivrant un extrait, un raffinat-2 et un raffinat intermédiaire. Le raffinat-2 est envoyé dans une isomérisation fonctionnant préférentiellement en phase liquide et à basse température. Le raffinat intermédiaire à teneur enrichie en éthylbenzène est isomérisé en phase vapeur.

De manière alternative, le brevet US8273934 décrit un procédé de production de paraxylène comprenant :
(a) une séparation d'une charge contenant des hydrocarbures en C8 pour produire un flux riche en hydrocarbures en C8,
(b) une séparation d'une première partie du flux riche en hydrocarbures en C8 pour produire un premier flux riche en paraxylène et un premier raffinat pauvre en paraxylène,
(c) une isomérisation d'au moins en partie du raffinat pour obtenir un isomérat,
(d) une séparation d'une deuxième partie du flux riche en hydrocarbures en C8 pour produire un deuxième flux riche en paraxylène et un deuxième raffinat pauvre en paraxylène,
(e) une isomérisation au moins partiellement en phase liquide d'au moins une partie du deuxième raffinat pour produire un isomérat,
(f) la récupération d'au moins une partie d'au moins un des deux flux riches en paraxylène pour faire du paraxylène à haute pureté via une séparation par adsorption,
(g) l'envoi d'au moins une partie du premier isomérat et d'au moins une partie du deuxième isomérat à l'unité de séparation (a).

US2014/155667 divulgue un procédé de production de paraxylène à haute pureté à partir d'une coupe xylènes contenant de l'éthylbenzène et des composés C9+.

Il est à noter que, lorsque l'unité de séparation du paraxylène est un procédé de séparation par adsorption, la teneur en C9+ doit être très faible dans la charge (typiquement inférieure à quelques centaines de ppm pds) afin d'éviter l'accumulation de ces composés dans la boucle de recyclage du solvant. Industriellement et dans les brevets ci-dessus, on a donc de manière systématique un traitement d'au moins une partie de la charge alimentant la ou les unité(s) de séparation par adsorption par distillation avec une spécification très sévère sur la teneur en C9-aros. Or, cette séparation est très couteuse en énergie. Il existe donc un besoin d'optimisation de l'enchainement des procédés au sein de la boucle C8-aromatique afin de limiter les coûts opératoires, notamment ceux liés à la séparation des C9-aromatiques. Une telle problématique se pose également lors du remodelage d'unités existantes afin d'augmenter la quantité de paraxylène produite. On appelle cette opération dégoulottage, terme qui sera utilisé dans la suite du texte.
Il s'agit alors de limiter au maximum, les coûts d'investissement liés aux modifications à apporter à la boucle C8-aromatique, ainsi que les coûts opératoires de fonctionnement de l'enchainement de procédés après remodelage.
La présente invention utilise deux séparations par adsorption en LMS, et deux isomérisations, combinée avec l'injection de la charge fraiche sur une seule des deux séparation par adsorption. Elle permet de limiter voire d'éliminer le traitement de la charge en amont d'une des unités de séparation, contrairement aux solutions proposées par l'art antérieur.
L'utilisation de deux unités de séparation par adsorption en LMS permet de plus de traiter plus de charge que l'enchaînement proposé dans le brevet FR2862638, ce qui permet de maximiser l'utilisation d'adsorbant de nouvelle génération très performant et de répondre aux besoins actuels de production de paraxylène supérieure à 1 million de tonnes par an sur un seul site.
La mutualisation des colonnes en aval des deux unités de séparation par adsorption permet également de ne pas multiplier les colonnes de distillation et de minimiser ainsi l'investissement. Ceci permet en outre une utilisation optimale des colonnes à distiller existantes dans le cas du remodelage d'une unité de séparation existante à 24 lits en deux unités de séparation à 12 lits. Enfin, le mélange des deux raffinats intermédiaires (enrichis en éthylbenzène) issus des deux unités de séparation par adsorption permet l'optimisation de la conversion de l'EB dans l'isomérisation en phase gazeuse.

Dans la suite du texte on parle d'unité de séparation pour désigner les unités de séparation en lit mobile simulé, d'adsorbeurs pour désigner des ensembles de lits d'adsorbant, une unité pouvant contenir un ou plusieurs adsorbeurs.

On parle d'unité d'isomérisation et de colonnes à distiller pour désigner les autres équipements du procédé.

### DESCRIPTION SOMMAIRE DES FIGURES

La figure 1 représente le schéma du procédé selon la présente invention avec les deux unités de séparation en lit mobile simulé notées SMB-1 et SMB-2 et les deux unités d'isomérisation notées ISOM-1 et ISOM-2.
La figure 2 représente une variante du procédé selon l'invention qui conduit à utiliser des colonnes à parois internes dite « divided wall column » dans la terminologie anglo-saxonne.
La figure 3 représente un schéma du procédé selon l'art antérieur qui est utilisée pour l'exemple 1.

### DESCRIPTION SOMMAIRE DE L'INVENTION

La présente invention peut se définir comme un procédé de production de paraxylène à haute pureté, à partir d'une coupe xylènes contenant de l'éthylbenzène et des composés en C9+, procédé utilisant deux unités de séparation en lit mobile simulé (SMB-1 et SMB-2) fonctionnant en série, et deux unités d'isomérisation (ISOM-1 et ISOM-2).

Par fonctionnement en série des unités de séparation en lit mobile simulé (SMB-1 et SMB-2), on entend que l'effluent de l'unité de séparation (SMB-1) est utilisée indirectement comme charge de l'unité de séparation (SMB-2), indirectement signifiant que des unités de séparation ou d'isomérisation peuvent s'intercaler sur le trajet de l'effluent de l'unité de séparation (SMB-1).

Plus précisément, le procédé selon la présente invention consiste en la suite d'étapes suivantes :
- on envoie la charge (2) dans une colonne de distillation (S-1) d'où l'on soutire en tête un mélange (3) comprenant la majeure partie du métaxylène, du paraxylène, de l'éthylbenzène, et au moins une partie de l'orthoxylène, et d'où l'on soutire en fond un flux (4) d'hydrocarbures en C9-C10 et la partie restante d'orthoxylène,
- on effectue une séparation du mélange de tête (3) dans l'unité de séparation (SMB-1) comportant au moins un adsorbeur contenant une pluralité de lits interconnectés et travaillant en boucle fermée, ledit adsorbeur comprenant au moins cinq zones délimitées par les injections du flux (3) et du désorbant (16), et les soutirages d'un premier extrait (5) contenant du paraxylène, d'un premier raffinat intermédiaire (7) contenant de l'éthylbenzène, et d'un premier raffinat-2 (8) contenant de l'orthoxylène et du métaxylène,
- on effectue une séparation dans l'unité de séparation (SMB-2) de l'isomérat (11) issu de l'unité d'isomérisation (ISOM-1), ladite unité de séparation (SMB-2) étant constituée d'au moins un adsorbeur contenant une pluralité de lits interconnectés et travaillant préférentiellement en boucle fermée, et ladite unité de séparation comprenant au moins cinq zones délimitées par les injections du flux (11) et du désorbant (17), et les soutirages d'un deuxième extrait (12) contenant du paraxylène, d'un deuxième raffinat intermédiaire (14) contenant de l'éthylbenzène, et d'un deuxième raffinat-2 (15) contenant de l'orthoxylène et du métaxylène,
- on distille le premier extrait (5) issu de la colonne (SMB-1) dans une colonne (EXT-1), pour récupérer une première fraction (6) enrichie en paraxylène,
- on mélange les deux raffinats-2, flux (8) et (15), pour former le flux (9) qui est distillé dans une colonne (RAF-2) de manière à éliminer sensiblement tout le désorbant et pour soutirer une première fraction distillée (10),
- on alimente par le flux (10) une première unité d'isomérisation (ISOM-1) pour obtenir le premier isomérat (11),
- on mélange les deux raffinats intermédiaires, flux (7) et (14), pour former le flux (18) qui est distillé dans une colonne (RAFINTER) pour éliminer sensiblement tout le désorbant et pour soutirer une deuxième fraction distillée (19),
- on alimente par le flux (19) une deuxième unité d'isomérisation (ISOM-2) pour obtenir un deuxième isomérat (20) qui est recyclé en entrée de la colonne de séparation (S-1).

La présente invention peut présenter plusieurs variantes dont certaines sont décrites ci-dessous.

Toutes ces variantes sont compatibles entre elles.

Selon une variante du procédé de production de paraxylène à haute pureté selon l'invention,
- on distille le premier extrait (5) issu de l'unité de séparation (SMB-1) dans une colonne (EXT-1), pour récupérer une première fraction (6) enrichie en paraxylène,
- on distille le deuxième extrait (12) dans une colonne (EXT-2), pour récupérer une deuxième fraction (13) enrichie en paraxylène.

Dans une autre variante du procédé de production de paraxylène à haute pureté selon l'invention, les deux extraits (5) et (12) sont distillés dans une seule colonne d'extrait commune pour récupérer une seule fraction enrichie en paraxylène.

Selon une variante préférée du procédé de production de paraxylène à haute pureté selon l'invention, l'unité d'isomérisation (ISOM1) fonctionne en phase liquide aux conditions suivantes :
- Température inférieure à 300°C, de préférence comprise entre 200 et 260°C,
- Pression inférieure à 4 MPa, de préférence comprise entre 2 et 3 MPa,
- Vitesse spatiale inférieure à 10 h⁻¹, de préférence comprise entre 2 h⁻¹ et 4 h⁻¹,
- Catalyseur comportant au moins une zéolithe présentant des canaux dont l'ouverture est définie par un anneau à 10 ou 12 atomes d'oxygène (10 MR ou 12 MR), préférentiellement un catalyseur comportant au moins une zéolithe présentant des canaux dont l'ouverture est définie par un anneau à 10 atomes d'oxygène (10 MR), et de manière encore plus préférée, un catalyseur comportant une zéolithe de type ZSM-5.

Selon une autre variante du procédé de production de paraxylène à haute pureté selon l'invention, l'unité d'isomérisation (ISOM-2) fonctionne en phase gazeuse aux conditions suivantes :
- température supérieure à 300°C, de préférence de 360°C à 480°C,
- pression inférieure à 2,5 MPa et de préférence de 0,5 à 0,8 MPa,
- vitesse spatiale inférieure à 10 h⁻¹, de préférence comprise entre 0,5 h⁻¹ et 6 h⁻¹,
- rapport molaire hydrogène sur hydrocarbure inférieur à 10, et de préférence compris entre 3 et 6.

Selon une autre variante du procédé de production de paraxylène à haute pureté selon l'invention, le catalyseur utilisé pour l'unité d'isomérisation ISOM-2 contient une zéolithe acide, par exemple de type structural MFI, MOR, MAZ, MTW, FAU et/ou EUO, et de manière préférée, contient une zéolithe de type structural EUO, et au moins un métal du groupe VIII de la classification périodique des éléments.

Selon une autre variante du procédé de production de paraxylène à haute pureté selon l'invention, le catalyseur utilisé pour l'unité d'isomérisation ISOM-2 comporte au moins une zéolithe présentant des canaux dont l'ouverture est définie par un anneau à 10 ou 12 atomes d'oxygène (10 MR ou 12 MR), et au moins un métal du groupe VIII de teneur comprise entre 0,1 et 0,3% poids, bornes incluses.

Selon une autre variante du procédé de production de paraxylène à haute pureté selon l'invention, l'unité de séparation (SMB-1) utilise comme désorbant le PDEB.

Dans une variante du procédé selon la présente invention, l'unité de séparation (SMB-2) utilise comme désorbant le toluène.

Dans une variante du procédé selon la présente invention, les unités de séparation (SMB-1) et (SMB-2) contiennent de 6 à 24 lits, et de manière préférée de 8 à 15 lits, répartis sur un ou plusieurs adsorbeurs, le nombre de lits étant ajusté de manière à ce que chaque lit ait de préférence une hauteur comprise entre 0,70 m et 1,40 m.

Dans une autre variante du procédé selon la présente invention, la répartition de la quantité de solide adsorbant dans les unités de séparation (SMB-1) et (SMB-2) est la suivante :
- la quantité de solide adsorbant en zone 1 est de 16%±5%,
- la quantité de solide adsorbant en zone 2 est de 40%±5%,
- la quantité de solide adsorbant en zone 3A est de 16%±5%,
- la quantité de solide adsorbant en zone 3B est de 16%±5%,
- la quantité de solide adsorbant en zone 4 est de 12%±5%,

Dans une autre variante du procédé de production de paraxylène à haute pureté selon l'invention, le rapport volumétrique désorbant sur charge est d'au plus 1,7/1 et de manière préférée compris entre 1,5/1 et 0,4/1, bornes comprises.

Dans une autre variante du procédé de production de paraxylène à haute pureté selon la présente invention, le rapport des débits de raffinat intermédiaire et de raffinat 2 est compris entre 0,3/1 et 5/1, et de manière préférée compris entre 0,4/1 et 1/1 bornes comprises.

Dans une autre variante du procédé de production de paraxylène à haute pureté selon l'invention, pour l'unité de séparation (SMB-2), le rapport volumétrique désorbant sur charge est d'au plus 1,7/1 et de manière préférée compris entre 1,5/1 et 0,4/1 bornes comprises.

Dans une autre variante du procédé de production de paraxylène à haute pureté à partir d'une coupe xylènes contenant de l'éthylbenzène et des composés en C9+ selon la revendication 1, dans lequel pour l'unité de séparation (SMB-2), le rapport des débits de raffinat intermédiaire et de raffinat 2 est compris entre 0,3/1 et 5/1, et de manière préférée compris entre 0,4/1 et 1/1 bornes comprises.

Dans une autre variante du procédé de production de paraxylène à haute pureté selon l'invention, on peut procéder au dégoulotage d'une unité de séparation existante, constituée de deux adsorbeurs en série, de la manière suivante :
- on connecte le dernier lit du premier adsorbeur au premier lit du premier adsorbeur via une ligne contenant au moins une pompe de recirculation, ce premier adsorbeur servant d'unité de séparation (SMB-1)
- on connecte le dernier lit du deuxième adsorbeur au premier lit du deuxième adsorbeur via une ligne contenant au moins une pompe de recirculation, ce deuxième adsorbeur servant d'unité de séparation (SMB-2).

De manière générale, la configuration des deux adsorbeurs peut être à nombre de lits fixe dans chacun des adsorbeurs, ou à nombre de lits fixe dans l'un des adsorbeurs et variable dans l'autre, ou encore variable pour les deux adsorbeurs.

De manière préférée, les deux adsorbeurs sont à nombre de lits fixes dans chacune des zones chromatographiques.

Enfin, dans une autre variante du procédé de production de paraxylène à haute pureté selon l'invention, la configuration des deux adsorbeurs en lit mobile simulé est à nombre de lits fixes dans chacune des zones chromatographiques de chacun des deux adsorbeurs.

### DESCRIPTION DETAILLEE DE L'INVENTION

On envoie la charge (2) dans une colonne de distillation (S-1) d'où l'on soutire en tête un mélange (3) comprenant la majeure partie du métaxylène, du paraxylène, de l'éthylbenzène, et au moins une partie de l'orthoxylène, et d'où l'on soutire en fond un flux (4) d'hydrocarbures en C9-C10 et la partie restante d'orthoxylène.

On effectue une première séparation du mélange de tête (3) dans l'unité de séparation (SMB-1) constituée d'une pluralité de lits interconnectés et travaillant en boucle fermée, ladite unité de séparation comprenant au moins cinq zones délimitées par les injections du flux (3) constituant la première charge de la colonne et du désorbant (16), et les soutirages d'un premier extrait (5) contenant du paraxylène, d'un premier raffinat intermédiaire (7) contenant de l'éthylbenzène, et d'un premier raffinat-2 (8) contenant de l'orthoxylène et du métaxylène.

Les cinq zones de l'unité de séparation (SMB-1) sont les suivantes :
- la zone 1 comprise entre l'injection du désorbant (16) et le soutirage de l'extrait (5),
- la zone 2 comprise entre le soutirage de l'extrait (5) et l'injection de la charge (3),
- la zone 3A comprise entre l'injection de la charge (3) et le soutirage du raffinat intermédiaire (7),
- la zone 3B comprise entre le soutirage du raffinat intermédiaire (7) et le soutirage du raffinat-2 (8),
- la zone 4 comprise entre le soutirage du raffinat-2 (8) et l'injection du désorbant (16).

On effectue une deuxième séparation en lit mobile simulé (SMB-2) de l'isomérat (11) issu de l'isomérisation (ISOM-1), dont on a optionnellement retiré tout ou partie des composés lourds en C9 et C10 par distillation (soit dans une colonne dédiée, soit dans la colonne S-1). De manière préférée, l'isomérat (11) est envoyé à l'unité de séparation en lit mobile simulé (SMB-2) sans étape intermédiaire d'élimination des composés en C9+. L'unité de séparation (SMB-2) est constituée d'une pluralité de lits interconnectés et travaillant en boucle fermée, ladite unité de séparation comprenant au moins cinq zones délimitées par les injections du flux (11) constituant la deuxième charge de la colonne et du désorbant (17), et les soutirages d'un deuxième extrait (12) contenant du paraxylène, d'un deuxième raffinat intermédiaire (14) contenant de l'éthylbenzène, et d'un deuxième raffinat-2 (15) contenant de l'orthoxylène et du métaxylène.

Les cinq zones de la colonne SMB-2 sont les suivantes :
- La zone 1 comprise entre l'injection du désorbant (17) et le soutirage de l'extrait (12),
- La zone 2 comprise entre le soutirage de l'extrait (12) et l'injection de la deuxième charge (11),
- La zone 3A comprise entre l'injection de la deuxième charge (11) et le soutirage du deuxième raffinat intermédiaire (14),
- La zone 3B comprise entre le soutirage du deuxième raffinat intermédiaire (14) et le soutirage du deuxième raffinat-2 (15),
- La zone 4 comprise entre le soutirage du deuxième raffinat-2 (15) et l'injection du désorbant (17).

On distille préférentiellement un premier extrait (5) dans une colonne de distillation (EXT-1), pour récupérer une première fraction (6) enrichie en paraxylène.

On distille préférentiellement un deuxième extrait (12) dans une colonne de distillation (EXT-2), pour récupérer une deuxième fraction (13) enrichie en paraxylène. Les deux extraits (5) et (12) peuvent également être distillés dans une seule colonne d'extrait commune pour récupérer une seule fraction enrichie en paraxylène.

Les deux raffinats-2 flux (8) et (15) sont préférentiellement mélangés pour former le flux (9) qui est distillé dans une colonne de distillation (RAF-2) pour éliminer sensiblement tout le désorbant et pour soutirer une première fraction distillée (9) pauvre en éthylbenzène.

Les deux raffinats-2 flux (8) et (15) peuvent également être distillés dans deux colonnes différentes, puis on mélange les deux fractions distillées pauvres en éthylbenzène. La ou les fraction(s) distillée(s) obtenue(s) alimente(nt) une première unité d'isomérisation (ISOM-1) pour obtenir un premier isomérat (11) alimentant préférentiellement l'unité de séparation (SMB-2), mais pouvant être en partie recyclé en entrée de la colonne de distillation (S-1).

Les deux raffinats intermédiaires flux (7) et (14) sont préférentiellement mélangés pour former le flux (18) et distillés dans une colonne (RAFINTER) pour éliminer sensiblement tout le désorbant et pour soutirer une deuxième fraction distillée (19) riche en éthylbenzène.

Les deux raffinats intermédiaires flux (7) et (14) peuvent également être distillés dans deux colonnes différentes puis on mélange les deux fractions distillées riches en éthylbenzène. La ou les fraction(s) distillée(s) obtenue(s) alimente(nt) une deuxième unité d'isomérisation (ISOM-2) pour obtenir un deuxième isomérat (20) recyclé en entrée de la colonne de séparation (S-1).

La première unité d'isomérisation (ISOM-1) travaillant de préférence en phase liquide, est généralement opérée dans les conditions suivantes :
- Température inférieure à 300°C, de préférence 200°C à 260°C
- Pression inférieure à 4 MPa, de préférence 2 à 3 MPa
- Vitesse spatiale horaire (VVH) inférieure à 10h⁻¹ (10 litres par litre et par heure), de préférence comprise entre 2 et 4h⁻¹.
- Catalyseur comportant au moins une zéolithe présentant des canaux dont l'ouverture est définie par un anneau à 10 ou 12 atomes d'oxygène (10 MR ou 12 MR), préférentiellement un catalyseur comportant au moins une zéolithe présentant des canaux dont l'ouverture est définie par un anneau à 10 atomes d'oxygène (10 MR), et de manière encore plus préférée, un catalyseur comportant une zéolithe de type ZSM-5.

La deuxième unité d'isomérisation (ISOM-2) travaillant en phase gazeuse est généralement opérée dans les conditions suivantes :
- Température supérieure à 300°C, de préférence 350°C à 480°C
- Pression inférieure à 4 MPa, de préférence comprise entre 0,5 et 2 MPa
- Vitesse spatiale horaire (VVH) inférieure à 10h⁻¹ (10 litres par litre et par heure), de préférence comprise entre 0,5 et 6 h⁻¹.
- Catalyseur comportant au moins une zéolithe présentant des canaux dont l'ouverture est définie par un anneau à 10 ou 12 atomes d'oxygène (10 MR ou 12 MR), préférentiellement un catalyseur comportant une zéolithe de type structural EUO, MTW ou MOR, et au moins un métal du groupe VIII,
- Rapport molaire H₂/hydrocarbures inférieur à 10, de préférence compris entre 3 et 6.

Le catalyseur de l'unité d'isomérisation en phase gazeuse (ISOM-2) peut comprendre une zéolithe de type structural EUO, MTW ou MOR, et au moins un métal du groupe VIII de la classification périodique des éléments dans un proportion pondérale de 0,01% à 2% par rapport au catalyseur. Le catalyseur de l'unité d'isomérisation en phase gazeuse (ISOM-2) peut contenir dans certains cas une zéolithe EU-1 et du platine.

Les désorbants utilisés dans les unités de séparation en lit mobile simulé (SMB-1 et SMB-2) sont généralement choisis parmi le paradiéthylbenzène, le toluène, le paradifluorobenzène ou des diéthylbenzènes en mélange.

Le rapport volumique du désorbant sur la charge dans les unités de séparation en lit mobile simulé (SMB-1 et SMB-2) est compris entre 0,5 et 2,5, et de préférence compris entre 0,8 et 2.

Les unités de séparation en lit mobile simulé (SMB-1 et SMB-2) sont opérées à une température comprise entre 20°C et 250°C, de préférence entre 90°C et 210°C, et de manière encore préférée entre 140°C et 180°C, et sous une pression comprise entre la pression de bulle de xylènes à la température opératoire et 2 MPa.

La teneur en éthylbenzène de la seconde fraction distillée issue du raffinat-2 (flux (8) et (14)) est inférieure à celle de la charge et atteint de préférence au plus 10% poids.

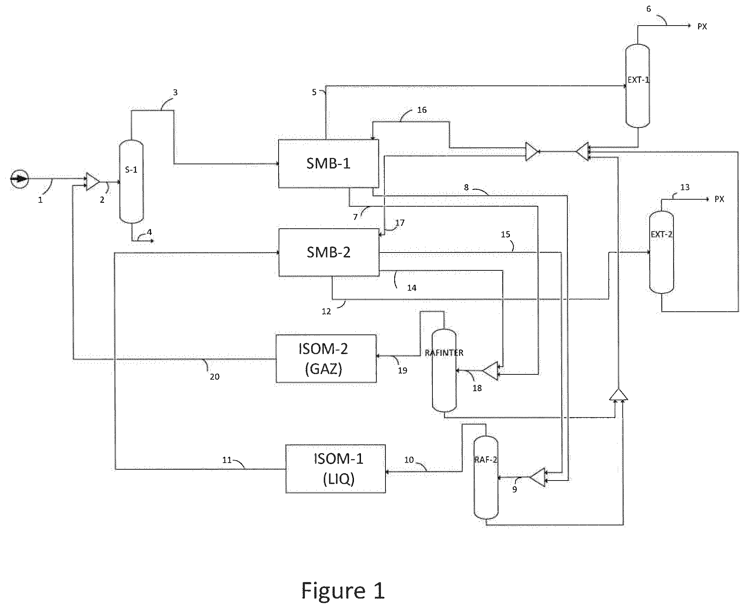

Le fonctionnement du procédé selon l'invention, et en particulier la composition des différents flux, est précisé ci-après avec référence à la figure 1.

La charge fraîche est introduite par la ligne (1) dans une colonne à distiller (S-1). Cette charge fraîche contient majoritairement des composés C8-aromatiques, xylènes et éthylbenzène, en proportion variable selon l'origine de la coupe. Elle peut contenir éventuellement des impuretés en quantité variable selon l'origine de la charge qui seront essentiellement des composés C9 et C10 aromatiques et des composés paraffiniques et naphténiques.

La teneur en impuretés naphténiques ou paraffiniques est avantageusement inférieure à 1 % poids. De manière préférée, cette teneur est inférieure à 0,3 % poids, et de manière encore préférée cette teneur est inférieure à 0,1 % poids.

La charge peut être issue, soit d'une unité de reformage, soit d'une unité de dismutation du toluène, soit d'une unité de transalkylation du toluène et des C9 aromatiques.

On ajoute à la charge fraîche un isomérat véhiculé par une ligne (20).

L'effluent de fond (4) de la colonne (S-1) est constitué essentiellement de composés en C9 et C10 aromatiques, et éventuellement d'orthoxylène.

Optionnellement, le mélange (4) d'orthoxylène et d'hydrocarbures aromatiques en C9-C10 soutiré en fond de la colonne de distillation (S1), peut être envoyé dans une autre colonne de distillation d'où l'on extrait en tête un flux d'orthoxylène de haute pureté (au moins 98,5 %), et en fond un flux contenant des hydrocarbures en C9-C10.

L'effluent de tête (3) de la colonne (S-1), constitue la charge d'une unité de séparation en lit mobile simulé (SMB-1). L'unité de séparation en lit mobile simulé (SMB-1) est alimentée d'une part par la charge véhiculée par la ligne (3), et d'autre part par du désorbant véhiculé par une ligne (16).

Tout type de désorbant peut être utilisé. Le désorbant préféré est le paradiéthylbenzène, cependant d'autres désorbants tels que le toluène, le paradifluorobenzène ou des diéthylbenzènes en mélange peuvent également convenir.

Les effluents de l'unité (SMB-1) sont un extrait (5), un raffinat intermédiaire (7) et un raffinat-2 noté (8), ladite unité de séparation comprenant au moins cinq zones délimitées par les injections de charge et de désorbant, et les soutirages de raffinat intermédiaire, de raffinat-2 et d'extrait.

Le nombre total de lits de l'unité de séparation (SMB-1) selon l'invention est de préférence compris entre 6 et 24 lits, et de manière encore plus préférée entre 8 et 15 lits répartis sur un ou plusieurs adsorbeurs.

Le nombre de lits sera ajusté de manière à ce que chaque lit ait de préférence une hauteur comprise entre 0,70 m et 1,40 m.

La répartition de la quantité de solide adsorbant dans chaque zone est la suivante :
- la quantité de solide adsorbant en zone 1 est de 16%±5%,
- la quantité de solide adsorbant en zone 2 est de 40%±5%,
- la quantité de solide adsorbant en zone 3A est de 16%±5%,
- la quantité de solide adsorbant en zone 3B est de 16%±5%,
- la quantité de solide adsorbant en zone 4 est de 12%±5%,

Selon une caractéristique préférée de l'invention, on peut injecter le désorbant et la charge dans l'unité de séparation (SMB-1) dans un rapport volumétrique désorbant sur charge d'au plus 1,7/1, et de manière préférée compris entre 1,5/1 et 0,4/1, bornes incluses.

Selon une caractéristique préférée de l'invention, le rapport des débits de raffinat intermédiaire et de raffinat 2 est compris entre 0,3/1 et 5/1 bornes incluses, et de manière préférée entre 0,4/1 et 1/1 bornes incluses.

L'extrait (5) est constitué essentiellement de toluène, de paraxylène et de désorbant.

Le raffinat intermédiaire (7) est constitué essentiellement de toluène, de métaxylène et d'orthoxylène, d'éthylbenzène, de paraxylène pour la partie non récupéré dans l'extrait, et de désorbant.

Le raffinat-2 (8) est constitué essentiellement de métaxylène et d'orthoxylène et de désorbant. Il est substantiellement exempt de paraxylène et appauvri en éthylbenzène. L'extrait (5) est envoyé dans une colonne à distiller (EXT-1).

On soutire de la colonne à distiller (EXT-1) le désorbant qui est renvoyé dans l'unité de séparation (SMB-1) par la ligne (16), et un mélange de paraxylène et de toluène par la ligne (6).

Le raffinat-2 (8) est envoyé dans une colonne à distiller (RAF-2).

On soutire de la colonne à distiller (RAF-2) du désorbant qui est renvoyé vers la ligne (16), et un mélange de métaxylène et d'orthoxylène par une ligne (9), que l'on envoie vers l'unité d'isomérisation (ISOM-1).

L'unité d'isomérisation (ISOM-1), préférentiellement en phase liquide, travaille préférentiellement dans les conditions suivantes:
- température inférieure à 300°C, de préférence comprise entre 200 et 260°C,
- pression inférieure à 4 MPa, de préférence comprise entre 2 et 3 MPa,
- vitesse spatiale inférieure à 10 h⁻¹, de préférence comprise entre 2 h⁻¹ et 4 h⁻¹.

Tous les catalyseurs susceptibles d'isomériser les hydrocarbures à 8 atomes de carbone conviennent pour l'unité d'isomérisation (ISOM-2) de la présente invention. De préférence, on utilisera un catalyseur contenant une zéolithe de type ZSM-5

L'effluent de l'unité d'isomérisation (ISOM-1) est renvoyé par la ligne (11), soit vers la colonne à distiller (S-1), soit directement à l'entrée de l'unité de séparation (SMB-2) dans le cas où la teneur en composés autres que les C8-aromatiques est très faible, typiquement de l'ordre de 1% poids. La teneur en C9 est typiquement inférieure à 1000 ppm poids.

Tout type de désorbant peut être utilisé. Le désorbant préféré est le paradiéthylbenzène, cependant d'autres désorbants tels que le toluène, le paradifluorobenzène ou des diéthylbenzènes en mélange peuvent également convenir.

Les effluents de l'unité de séparation (SMB-2) sont un extrait (12), un raffinat intermédiaire (14) et un raffinat-2 (15), ladite unité de séparation comprenant au moins cinq zones délimitées par les injections de charge et de désorbant, et les soutirages de raffinat intermédiaire, de raffinat-2 et d'extrait. Le nombre total de lits de l'unité de séparation (SMB-2) selon l'invention est de préférence compris entre 6 et 24 lits, et de manière encore plus préférée compris entre 8 et 15 lits répartis sur un ou plusieurs adsorbeurs.

Le nombre de lits sera ajusté de manière à ce que chaque lit ait de préférence une hauteur comprise entre 0,70 m et 1,40 m.

La répartition de la quantité de solide adsorbant dans chaque zone est la suivante :
- la quantité de solide adsorbant en zone 1 est de 16%±5%,
- la quantité de solide adsorbant en zone 2 est de 40%±5%,
- la quantité de solide adsorbant en zone 3A est de 16%±5%,
- la quantité de solide adsorbant en zone 3B est de 16%±5%,
- la quantité de solide adsorbant en zone 4 est de 12%±5%,

Selon une autre caractéristique préférée de l'invention, on peut injecter le désorbant et la charge dans l'unité de séparation(SMB-2) dans un rapport volumétrique désorbant sur charge d'au plus 1,7/1, et de manière préférée compris entre 1,5/1 et 0,4/1, bornes incluses.

Selon une caractéristique préférée de l'invention, le rapport des débits de raffinat intermédiaire et de raffinat 2 est compris entre 0,3/1 et 5/1 bornes incluses, et de manière préférée, entre 0,4/1 et 1/1 bornes incluses.

La configuration (nombre moyen de lits par zone) des deux unités en lit mobile simulé (SMB-1 et SMB-2) peut être :
- à nombre de lits fixes dans chacune des zones chromatographiques (mode « lit mobile simulé » tel que défini dans le brevet FR 2 976 501),
- à nombre de lits variable pour un adsorbeur (mode « VARICOL » tel que défini dans le brevet FR 2 976 501) et fixe pour l'autre,
- à nombre de lits variable pour les deux adsorbeurs.

L'extrait (12) est constitué essentiellement de toluène, de paraxylène et de désorbant.

Le raffinat intermédiaire (14) est constitué essentiellement de toluène, de métaxylène et d'orthoxylène, d'éthylbenzène, de paraxylène pour la partie non récupéré dans l'extrait, et de désorbant.

Le raffinat-2 (15) est constitué essentiellement de métaxylène et d'orthoxylène et de désorbant. Il est substantiellement exempt de paraxylène et appauvri en éthylbenzène. L'extrait (12) est envoyé dans une colonne à distiller (EXT-2).

On soutire de la colonne à distiller (EXT-2) le désorbant qui est renvoyé dans la zone 1 de la colonne (SMB-2) par la ligne (17) et un mélange de paraxylène et de toluène par la ligne (13).

Le raffinat intermédiaire est envoyé par une ligne (14) avec le raffinat intermédiaire (7) dans la colonne à distiller (RAFINTER). On soutire de la colonne à distiller (RAFINTER) du désorbant qui est réintroduit par la ligne (17) dans l'unité de séparation (SMB-2), et un mélange de xylènes et d'éthylbenzène par une ligne (19). Les effluents de la ligne (19) sont envoyés vers l'unité d'isomérisation (ISOM-2) fonctionnant à haute température en phase vapeur.

L'unité d'isomérisation (ISOM-2) est opérée de préférence dans les conditions suivantes :
- température supérieure à 300°C, de préférence de 360°C à 480°C,
- pression inférieure à 2,5 MPa, et de préférence de 0,5 à 0,8 MPa,
- vitesse spatiale inférieure à 10h⁻¹ de préférence comprise entre 0,5h⁻¹ et 6 h⁻¹,
- rapport molaire hydrogène sur hydrocarbure inférieur à 10, et de préférence compris entre 3 et 6.

Tous les catalyseurs susceptibles d'isomériser les hydrocarbures à 8 atomes de carbone, zéolithiques ou non, conviennent pour l'unité d'isomérisation (ISOM-2) de la présente invention. De préférence, on utilise un catalyseur contenant une zéolithe acide, par exemple de type structural MFI, MOR, MAZ, FAU et/ou EUO. De manière encore plus préférée, on utilise un catalyseur contenant une zéolithe de type structural EUO et au moins un métal du groupe VIII de la classification périodique des éléments.

De manière préférée, le catalyseur de l'unité d'isomérisation (ISOM-2) renferme de 1 à 70% poids d'une zéolithe de type structural EUO (EU-1 par exemple) comprenant du silicium et au moins un élément T choisi de préférence parmi l'aluminium et le bore dont le rapport Si/T est compris entre 5 et 100. La dite zéolithe est sous forme hydrogène au moins en partie, et la teneur en sodium est telle que le ratio atomique Na/T est inférieur à 0,1. Eventuellement le catalyseur de l'unité d'isomérisation peut contenir entre 0,01 et 2% poids d'étain ou d'indium, et du soufre à raison de 0,5 à 2 atomes par atome de métal du groupe VIII.

L'effluent de l'unité d'isomérisation (ISOM-2) est envoyé dans un train de séparations qui permet de récupérer une partie de l'hydrogène que l'on recycle à l'unité d'isomérisation (ISOM-2). La partie d'hydrogène non recyclé est compensée par un appoint d'hydrogène frais. On récupère à l'issue du train de séparation un isomérat constitué des fractions les plus lourdes qui est renvoyé vers la colonne à distiller (S-1) par la ligne (20).

Le procédé selon l'invention est particulièrement bien adapté à une modification d'une unité existante en vue d'une augmentation de la quantité de paraxylène produite, opération appelée dégoulottage.

Dans le cas d'un dégoulottage d'une boucle aromatique existante, l'invention consiste à augmenter de manière significative le débit de charge fraîche et le débit de paraxylène produit tout en continuant à utiliser les équipements principaux de la boucle soit :
1) la colonne de distillation des xylènes (S-1)
2) l'unité de séparation des xylènes en lit mobile simulé fonctionnant typiquement en 24 lits
3) l'unité d'isomérisation (ISOM-2) alimenté par le raffinat soutiré de la colonne de séparation convertissant l'éthylbenzène utilisant par exemple un catalyseur à base de zéolithe de type structural EUO comportant un réacteur, un compresseur de recyclage, une colonne de stabilisation et une colonne permettant de récupérer les naphtènes en C8 et C9 de manière à les recycler à la charge,
4) la colonne de raffinat (RAFINTER)
5) la colonne d'extrait (EXT-1)

Pour réaliser ce dégoulottage selon l'invention, on transforme un lit mobile simule à 24 lits contenant deux adsorbeurs de 12 lits en série en un procédé en deux adsorbeurs de douze lits chacun connecté en parallèle. Pour se faire :
- On connecte le douzième lit du premier adsorbeur au premier lit dudit premier adsorbeur via une ligne contenant au moins une pompe de recirculation,
- on connecte le douzième lit du deuxième adsorbeur au premier lit dudit deuxième adsorbeur via une ligne contenant au moins une pompe de recirculation.

Le système de contrôle et de régulation des débits d'injection de charge et de désorbant et des débits de soutirage de l'extrait et du raffinat de l'étape d'adsorption en 24 lits est adapté de manière à pouvoir gérer indépendamment les débits d'injection et de soutirage dans chacun des deux adsorbeurs du procédé remodelé selon l'invention.

Pour les dispositifs d'injection, cette opération pourra être réalisée soit en doublant le système pompe + organe de mesure pour réguler le débit injecté dans chacun des adsorbeurs, soit, dans le but de minimiser les coûts, en utilisant la pompe et l'organe de mesure préexistants qui gèreront l'ensemble des deux flux à injecter et en ajoutant un système de mesure et de régulation du débit alimentant l'un des deux adsorbeurs.

Lorsque l'alimentation ou le soutirage des fluides sur l'ensemble des plateaux du procédé existant en 24 lits est assurée par une pluralité de vannes commandées tout ou rien, il n'y a pas de modifications supplémentaires à apporter aux réseaux d'alimentation et de soutirage.

Lorsque l'alimentation ou le soutirage des fluides sur l'ensemble des plateaux du procédé existant en 24 lits est assurée par l'emploi d'une vanne rotative multi-voies, ces fonctions seront de manière préférée assurées par l'utilisation de deux vannes rotatives multi-voies 15 (en recyclant éventuellement la vanne préexistante sur l'une des deux voies après adaptation).

Dans le cas d'une unité existante en 24 lits constituée de deux fois douze lits en série, le flux principal circule du fond du premier adsorbeur vers la tête du second adsorbeur, et du fond du second adsorbeur vers la tête du premier adsorbeur.

Les flux issus des fonds des deux adsorbeurs sont alors réorientés pour circuler vers la tête de l'adsorbeur dont ils sont issus en réalisant les modifications de vannes et de conduites. Le flux de fond du premier adsorbeur est recyclé vers la tête dudit adsorbeur et le flux de fond du deuxième adsorbeur est recyclé vers la tête dudit adsorbeur.

La configuration (nombre moyen de lits par zone) des deux adsorbeurs peut être selon l'une des 3 variantes exposées plus haut c'est-à-dire :
- A nombre de lits fixes dans chacune des zones chromatographiques pour les deux adsorbeurs,
- à nombre de lits variable pour un adsorbeur et fixe pour l'autre,
- à nombre de lits variable pour les deux adsorbeurs.

De manière à séparer les raffinat-2 du désorbant, une nouvelle colonne à distiller (RAF-2) devra également être mise en place. La tête de la colonne à distiller (RAF-2) sera isomérisée dans l'unité d'isomérisation préférentiellement en phase liquide (ISOM-1) telle que décrite plus haut.

On ajoute une deuxième unité d'isomérisation en phase liquide (ISOM-1) dont l'effluent alimentera le lit mobile simulé (SMB-2) préférentiellement sans passer par la colonne de séparation (S-1) pour éviter l'ajout d'une deuxième colonne de séparation des xylènes.

D'autre part, il faut rajouter une colonne d'extrait (EXT-2) alimentée par l'extrait (12) de l'unité de séparation (SMB-2).

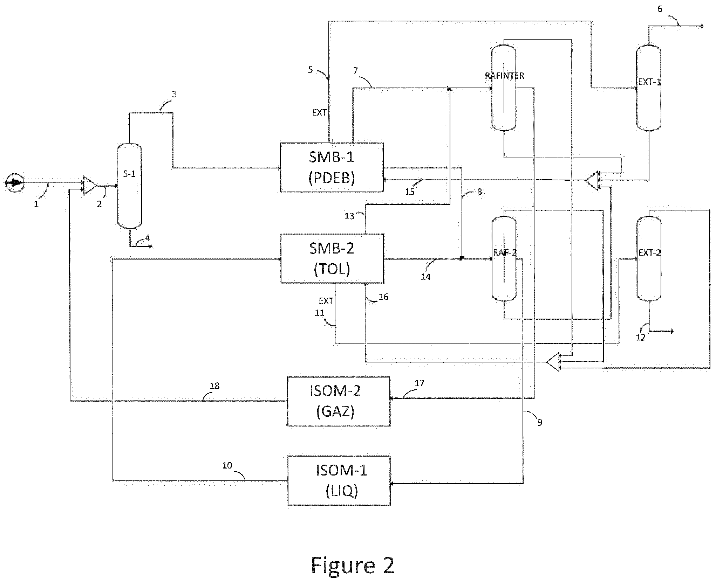

La figure 2 représente une variante du schéma de procédé selon l'invention qui diffère de celui de la figure 1 par le fait que l'unité de séparation (SMB-1) utilise le PDEB comme désorbant et l'unité de séparation (SMB-2) le toluène. Les flux raffinat-intermédiaire (flux (7) et (14)) sont mélangés avant l'entrée de la colonne à distiller (RAFINTER). Pour sortir le toluène en tête de la colonne à distiller (RAFINTER), le PDEB en fond et le raffinat-intermédiaire débarrassé de ces désorbants sur le côté, on a besoin d'une colonne à division longitudinale dite colonne à parois internes (« dividing wall column » dans la terminologie anglo-saxonne).

Une colonne de distillation à parois internes permet de séparer 3 composés à haute pureté via une seule colonne.

Il en est optionnellement de même pour la colonne à distiller (RAF-2) qui est alimentée par les flux raffinat-2 (flux (8) et (14)). Dans le cas où la colonne à distiller n'est pas du type à paroi interne, seul le paradiéthylbenzène est sorti en fond de la colonne pour être recyclé, le toluène étant envoyé à l'isomérisation avec la coupe C8-aromatiques.

Le fait d'utiliser deux désorbants différents dans les deux unités de séparation (SMB-1 et SMB-2) présente l'avantage d'éviter l'accumulation des impuretés aromatiques telles que le benzène et les composés aromatiques lourds en C9 et C10.

Utiliser deux désorbants différents dans les deux unités de séparation (SMB-1 et SMB-2) permet également de réaliser une intégration thermique entre les colonnes à distiller (EXT-1 et EXT-2). En effet, la tête de colonne à distiller (EXT-1) peut potentiellement rebouillir tout ou partie de la colonne à distiller (EXT-2).

### EXEMPLES

### Exemple 1 selon l'art antérieur :

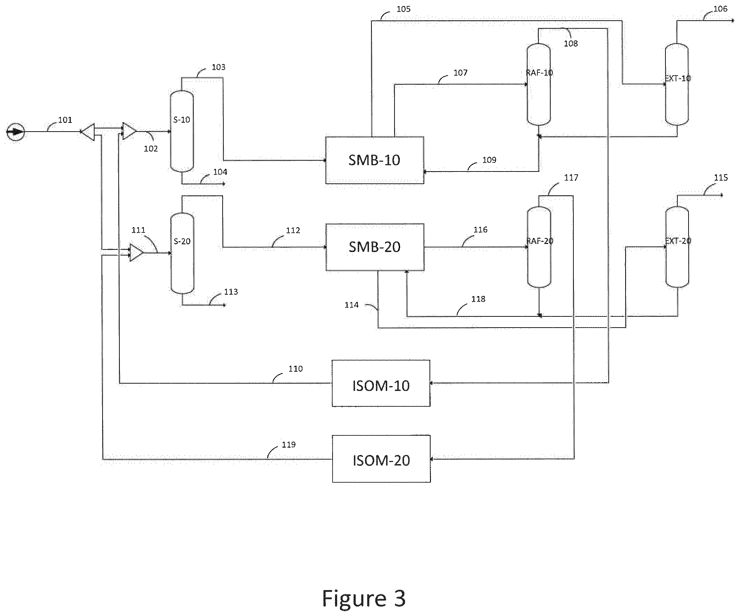

Cet exemple illustre l'art antérieur et décrit un complexe aromatique constitué de deux boucles C8-aromatique en parallèle, typiques des complexes industriels où la quantité de paraxylène produite est supérieure à la capacité acceptable par une boucle C8-aromatique unique, tel que schématisé sur la figure 3 et comportant :
- deux colonne des xylènes (S-10 et S-20) permettant d'extraire les aromatiques en C9 et C10 (flux 104 et 113) et d'envoyer aux unités d'adsorption (SMB-10 et SMB-20) un flux (103) et un flux (112) constitué essentiellement d'aromatiques en C8,
- une première unité d'adsorption en lit mobile simulé (SMB-10) à 4 zones de laquelle on soutire un extrait (105) et un unique raffinat (107),
- une première unité d'isomérisation (ISOM-10) alimentée par une partie (108) du raffinat (107) après élimination du désorbant (109) au moyen de la colonne à distiller (RAF-10),
- une première colonne d'extrait du paraxylène (EXT-10) dont on soutire en fond le désorbant qu'on recycle à l'adsorption (SMB-1) via le flux (109) et en tête une coupe riche en paraxylène (106),
- une deuxième unité d'adsorption en lit mobile simulé (SMB-20) à 4 zones de laquelle on soutire un extrait (114) et un unique raffinat (116),
- une deuxième unité d'isomérisation (ISOM-20) alimentée par une partie (117) du raffinat (116) après élimination du désorbant (118) au moyen de la colonne à distiller (RAF-20),
- une deuxième colonne d'extrait du paraxylène (EXT-20) dont on soutire en fond le désorbant qu'on recycle à l'adsorption (SMB-2) via le flux (118) et en tête une coupe riche en paraxylène (115).

Le bilan matière du procédé est décrit dans le tableau 1 ci-dessous.

Seuls les composés C8-aromatiques et C9+ sont décrits en négligeant les autres composés et la formation de C9+ dans les unités d'isomérisation. On utilise comme unité de débit le millier de tonnes par an (kt/an).

**TABLEAU 1**

| | | PX | EB | MOX | C9+ | Total |
|---|---|---|---|---|---|---|
| Charge fraîche | 101 | 23,6 | 15,6 | 67,7 | 13,8 | 120,6 |
| Charge S-10 | 102 | 50,0 | 22,9 | 148,5 | 6,9 | 228,3 |
| Charge SMB-10 | 103 | 50,0 | 22,9 | 148,5 | 0 | 221,4 |
| Fond S-10 | 104 | 0 | 0 | 0 | 6,9 | 6,9 |
| Tête EXT-10 | 106 | 50,0 | 0 | 0 | 0 | 50,0 |
| Entré ISOM-10 | 108 | 0 | 22,9 | 148,5 | 0 | 171,4 |
| Sortie ISOM-10 | 110 | 38,2 | 15,1 | 114,7 | 0 | 168,0 |
| Charge S-20 | 111 | 50,0 | 22,9 | 148,5 | 6,9 | 228,3 |
| Charge SMB-20 | 112 | 50,0 | 22,9 | 148,5 | 0 | 221,4 |
| Fond S-20 | 113 | 0 | 0 | 0 | 6,9 | 6,9 |
| Tête EXT-20 | 115 | 50,0 | 0 | 0 | 0 | 50,0 |
| Entré ISOM-20 | 117 | 0 | 22,9 | 148,5 | 0 | 171,4 |
| Sortie ISOM-20 | 119 | 38,2 | 15,1 | 114,7 | 0 | 168,0 |

La charge (101) qui alimente la boucle aromatique (mélange du réformat lourd et du fond de la colonne de toluène) présente un débit de 120,6 kt/an. Cette charge est divisée en deux flux égaux de 60,3 kt/an. On adjoint à une première partie de la charge (101) 168 kt/an d'isomérat (110) recyclés de l'unité d'isomérisation (ISOM-10) de manière à isomériser l'éthylbenzène. Le flux résultant (102) est distillé dans la colonne de xylènes (S-10).

On soutire en fond de la colonne (S-10) 6,9 kt/an d'un mélange de C9 et C10 aromatiques (104) et en tête 221,4 kt/an d'une coupe C8 aromatiques (103) dont la teneur en paraxylène est de 22.6%, la teneur en éthylbenzène est de 10,3%, la teneur en orthoxylène et métaxylène est de 67,1%.

Cette coupe est envoyée dans une unité d'adsorption en lit mobile simulé à quatre zones (SMB-10) et quatre flux principaux : la charge (103), le désorbant (109), l'extrait (105) et le raffinat (107). Cette unité est composée de 12 lits contenant une zéolithe X échangée au baryum. La température est de 175°C. La configuration est : 2 lits en zone 1, 5 lits en zone 2, 3 lits en zone 3 et 2 lits en zone 4. Le solvant utilisé est le paradiéthylbenzène.
L'extrait (105) en sortie de l'unité d'adsorption (SMB-10) est envoyé dans une colonne à distiller (EXT-10) de laquelle on tire en fond le désorbant recyclé vers l'unité d'adsorption (SMB-10), et en tête 50 kt/an d'un mélange (106), essentiellement constitué de toluène et de paraxylène.
Le raffinat est envoyé dans une colonne à distiller (RAF-10) de laquelle on tire en fond le désorbant recyclé vers l'unité d'adsorption (SMB-10), et en tête 171,4 kt/an d'un mélange (108).

Ce flux est envoyé dans une unité d'isomérisation (ISOM-10).
L'unité d'isomérisation (ISOM-10) travaille en phase gazeuse aux conditions suivantes :
Température : 385°C
Catalyseur : contient du platine et de la zéolithe EU-1
Vitesse spatiale : 3,5h⁻¹
Rapport H2/hydrocarbures : 4,4 :1
Pression : 0,9 MPa

La teneur en éthylbenzène du mélange introduit dans l'unité d'isomérisation (ISOM-10) est de 13,4%. On observe 2% de perte par craquage dans cette isomérisation soit un débit de 3,4 kt/an. L'éthylbenzène est isomérisé en partie, il en reste 9% dans le flux de sortie (110).

Cet isomérat (110) présente un débit de 168 kt/an, il est recyclé en entrée de la colonne S-10 où il est mélangé avec une partie de la charge fraîche (101) qui présente un débit de 60,3 kt/an.

On adjoint à une première partie de la charge (101) 168 kt/an d'isomérat (119) recyclés de l'unité d'isomérisation (ISOM-20) de manière à isomériser l'éthylbenzène. Le flux résultant (111) est distillé dans la colonne de xylènes (S-20).

On soutire en fond de la colonne (S-20) 6,9 kt/an d'un mélange de C9 et C10 aromatiques (flux 113) et en tête 221,4 kt/an de coupe C8 aromatiques (flux 112) dont la teneur en paraxylène est de 22,6%, et la teneur en éthylbenzène de 10,4%.

Cette coupe est envoyée dans une unité d'adsorption en lit mobile simulé à quatre zones (SMB-20) et quatre flux principaux : la charge (flux 112), le désorbant (flux 118), l'extrait (flux 114) et le raffinat (flux 116). Cette unité est composée de 12 lits contenant une zéolithe X échangée au baryum. La température est de 175°C. La configuration est : 2 lits en zone 1, 5 lits en zone 2, 3 lits en zone 3 et 2 lits en zone 4. Le solvant utilisé est le paradiéthylbenzène.

L'extrait (114) en sortie de l'unité d'adsorption (SMB-20) est envoyé dans une colonne à distiller (EXT-20) de laquelle on tire en fond le désorbant recyclé vers l'unité d'adsorption (SMB-20), et en tête 50 kt/an d'un mélange (115) essentiellement constitué de toluène et de paraxylène.

Le raffinat est envoyé dans une colonne à distiller (RAF-20) de laquelle on tire en fond le désorbant recyclé vers l'unité d'adsorption (SMB-20), et en tête 171,4 kt/an d'un mélange (117).

Ce flux est envoyé dans une unité d'isomérisation (ISOM-20). L'isomérat obtenu (119) est recyclé en entrée de la colonne S-20 où il est mélangé avec une partie de la charge fraîche (101).

L'unité d'isomérisation (ISOM-20) travaille en phase gazeuse aux conditions suivantes :
Température : 385°C
Catalyseur : contient du platine et de la zéolithe EU-1
Vitesse spatiale : 3,5h⁻¹
Rapport H2/hydrocarbures : 4,4 :1
Pression : 0,9 MPa

La teneur en éthylbenzène du mélange introduit dans l'unité d'isomérisation (ISOM-20) est de 13,4%.

On observe 2% de perte par craquage dans cette isomérisation soit un débit de 3,4 kt/an. L'éthylbenzène est isomérisé en partie. Il en reste 9% dans le flux de sortie (119).

Cet isomérat (119) présente un débit de 168 kt/an. Il est recyclé en entrée de la colonne S-20 où il est mélangé avec une partie de la charge fraîche (101) qui présente un débit de 60,3 kt/an.

### Exemple 2 selon l'invention :

Cet exemple illustre le procédé selon l'invention et décrit la boucle aromatique schématisée sur la figure 1 et comportant :
- une colonne des xylènes (S-1) permettant d'extraire les aromatiques en C9 et C10 (4) et d'envoyer à l'unité d'adsorption (SMB-1) un flux (3) constitué essentiellement d'aromatiques en C8,
- une première unité d'adsorption en lit mobile simulé (SMB-1) à 5 zones de laquelle on soutire un extrait (5), un raffinat intermédiaire (7) et un raffinat-2 (8),
- une première colonne d'extrait du paraxylène (EXT-1) dont on soutire en fond le désorbant qu'on recycle aux unités d'adsorption (SMB-1 et SMB-2) via les flux (16) et (17) et en tête une coupe riche en paraxylène (flux 6),
- une deuxième unité d'adsorption en lit mobile simulé (SMB-2) à 5 zones de laquelle on soutire un extrait (12), un raffinat intermédiaire (14) et un raffinat-2 (15),
- une deuxième colonne d'extrait du paraxylène (EXT-2) dont on soutire en fond le désorbant qu'on recycle aux unités d'adsorption (SMB-1 et SMB-2) via les flux (16) et (17) et en tête une coupe riche en paraxylène (flux 13),
- une première unité d'isomérisation (ISOM-1) alimentée par une partie (10) du raffinat-2 (9) constitué par le mélange des flux (8) et (15) après élimination du désorbant au moyen de la colonne à distiller (RAF-2),
- une deuxième unité d'isomérisation (ISOM-2) alimentée par une partie (19) du raffinat intermédiaire (18) constitué par le mélange des flux (7) et (14) après élimination du désorbant au moyen de la colonne à distiller (RAFINTER).

Le bilan matière du procédé est décrit dans le tableau 2 ci-dessous. Seuls les composés C8-aromatiques et C9+ sont décrits, on néglige les autres composés et la formation de C9+ dans les unités d'isomérisation. On utilise comme unité de débit le millier de tonnes par an (kt/an).

**TABLEAU 2**

| | | PX | EB | MOX | C9+ | Total |
|---|---|---|---|---|---|---|
| Charge fraîche | 1 | 22,6 | 15,2 | 64,7 | 13,2 | 115,8 |
| Charge S-1 | 2 | 51,1 | 26,5 | 150,4 | 13,2 | 241,2 |
| Charge SMB-1 | 3 | 51,1 | 26,5 | 150,4 | 0 | 228,0 |
| Fond S-1 | 4 | 0 | 0 | 0 | 13,2 | 13,2 |
| Tête EXT-1 | 6 | 51,1 | 0 | 0 | 0 | 51,1 |
| RAFF INTER | 7 | 0 | 15,9 | 50,4 | 0 | 66,3 |
| RAFF- 2 | 8 | 0 | 10,6 | 100,0 | 0 | 110,6 |
| Entrée ISOM LIQ | 10 | 0 | 17,7 | 195,6 | 0 | 213,3 |
| Sortie ISOM LIQ | 11 | 48,9 | 17,7 | 146,7 | 0 | 213,3 |
| Tête EXT-2 | 13 | 48,9 | 0 | 0 | 0 | 48,9 |
| RAFF-INTER | 14 | 0 | 10,6 | 51,0 | 0 | 61,7 |
| RAFF-2 | 15 | 0 | 7,1 | 95,7 | 0 | 102,8 |
| Entrée ISOM GAZ | 19 | 0 | 26,5 | 101,5 | 0 | 128,0 |
| Sortie ISOM GAZ | 20 | 28,5 | 11,3 | 85,6 | 0 | 125,4 |

La charge fraîche (1) qui alimente la boucle aromatique présente un débit de 115,8 kt/an.

On adjoint à cette charge 125,4 kt/an d'isomérat (20) recyclés de l'unité d'isomérisation (ISOM-2) isomérisant l'éthylbenzène. Le flux résultant (2) est distillé dans la colonne de xylènes (S-1).

On soutire en fond de la colonne (S-1) 13,2 kt/an d'un mélange de C9 et C10 aromatiques (4), et, en tête, 228 kt/an de coupe C8 aromatiques (3) dont la teneur en paraxylène est environ 22,4%, la teneur en éthylbenzène environ 11,6%, la teneur en orthoxylène et métaxylène environ 65,9%.

Cette coupe est envoyée dans une unité d'adsorption en lit mobile simulé à cinq zones (SMB-1) et cinq flux principaux : la charge (3), le désorbant (16), l'extrait (5), le raffinat intermédiaire (7) et le raffinat-2 (8). Cette unité est composée de 12 lits contenant une zéolithe X échangée au baryum. La température est de 175°C. La configuration est : 2 lits en zone 1, 5 lits en zone 2, 2 lits en zone 3A, 2 lits en zone 3B et un lit en zone 4. Le solvant utilisé est le paradiéthylbenzène.

L'extrait (5) en sortie de l'unité d'adsorption (SMB-1) est envoyé dans une colonne à distiller (EXT-1) de laquelle on tire en fond le désorbant recyclé vers l'unité d'adsorption (SMB-1), et en tête 51,1 kt/an d'un mélange (6) essentiellement constitué de toluène et de paraxylène.

L'isomérat (11) issu de l'unité d'isomérisation (ISOM-1) alimente une deuxième unité d'adsorption en lit mobile simulé à cinq zones (SMB-2) et cinq flux principaux : la charge (11), le désorbant (17), l'extrait (12), le raffinat intermédiaire (14) et le raffinat-2 (15). Cette unité est composée de 12 lits contenant une zéolithe X échangée au baryum.

La température est de 175°C.

La configuration est : 2 lits en zone 1, 5 lits en zone 2, 2 lits en zone 3A, 2 lits en zone 3B et un lit en zone 4. Le solvant utilisé est le paradiéthylbenzène.

L'extrait (12) en sortie de l'unité d'adsorption (SMB-2) est envoyé dans une colonne à distiller (EXT-2) de laquelle on tire en fond le désorbant recyclé vers l'unité d'adsorption (SMB-2), et en tête 48,9 kt/an d'un mélange (13) essentiellement constitué de toluène et de paraxylène.

Les raffinat-2 notés (8) et (15), sont mélangés et envoyés dans une colonne à distiller (RAF-2) de laquelle on tire en fond le désorbant recyclé vers les unités d'adsorption (SMB-1 et SMB-2), et en tête 213,3 kt/an d'un mélange (10).

Ce flux est envoyé dans une unité d'isomérisation (ISOM-1).

L'unité d'isomérisation (ISOM-1) travaille en phase liquide aux conditions suivantes :
Température : 240°C
Catalyseur : contient de la zéolithe ZSM-5
Vitesse spatiale : 3h⁻¹
Pression : 1,9 MPa

La teneur en éthylbenzène du mélange introduit dans l'unité d'isomérisation (ISOM-1) est environ 8,3%. L'éthylbenzène n'est pas converti, sa quantité est donc la même dans le flux de sortie (11). Cet isomérat (11) présente un débit de 213,3 kt/an, il est recyclé en entrée de l'unité d'adsorption (SMB-2) sans passer par la colonne (S-1).

Les raffinat intermédiaires (7) et (14) sont mélangés et envoyés dans une colonne à distiller (RAFINTER) de laquelle on tire en fond le désorbant recyclé vers les unités d'adsorption (SMB-1 et SMB-2), et en tête 128 kt/an d'un mélange (19).

Ce flux est envoyé dans une unité d'isomérisation (ISOM-2).

L'unité d'isomérisation (ISOM-2) travaille en phase gaz aux conditions suivantes :
Température : 385°C
Catalyseur : contient du platine et de la zéolithe EU-1
Vitesse spatiale : 3,5 h⁻¹
Pression : 0,9 MPa

La teneur en éthylbenzène du mélange introduit dans l'unité d'isomérisation (ISOM-2) est de 20,7%. On observe 2% de perte par craquage dans cette isomérisation soit un débit de 2,6 kt/an. L'éthylbenzène est isomérisé en partie, il en reste 9% dans le flux de sortie (20).

Cet isomérat (20) présente un débit de 125,4 kt/an, il est recyclé en entrée de la colonne S-1 où il est mélangé avec la charge fraîche (1) qui présente un débit de 115,8 kt/an.

L'invention présente plusieurs avantages par rapport à l'art antérieur. Tout d'abord l'isomérisation en phase liquide est moins consommatrice d'énergie que l'isomérisation en phase gaz. En effet, elle travaille à température plus faible. Elle travaille également sans recycle d'hydrogène, donc sans compresseur de recycle. Enfin, elle produit beaucoup moins de sous-produits, notamment des aromatiques en C9 ce qui permet de court-circuiter la colonne d'élimination des aromatiques en C9 (S-1) induisant une très forte baisse de l'énergie nécessaire à cette séparation.

## Revendications

1. Procédé de production de paraxylène à haute pureté à partir d'une coupe xylènes contenant de l'éthylbenzène et des composés en C9+, procédé utilisant deux unités de séparation en lit mobile simulé (SMB-1 et SMB-2), et deux unités d'isomérisation (ISOM-1 et ISOM-2) consistant en la suite d'étapes suivantes :
- on envoie la charge (2) dans une colonne de distillation (S-1) d'où l'on soutire en tête un mélange (3) comprenant la majeure partie du métaxylène, du paraxylène, de l'éthylbenzène, et au moins une partie de l'orthoxylène, et d'où l'on soutire en fond un flux (4) d'hydrocarbures en C9-C10 et la partie restante d'orthoxylène,
- on effectue une séparation du mélange de tête (3) dans l'unité de séparation (SMB-1) comportant au moins un adsorbeur contenant une pluralité de lits interconnectés et travaillant en boucle fermée, ledit adsorbeur comprenant au moins cinq zones délimitées par les injections du flux (3) et du désorbant (16), et les soutirages d'un premier extrait (5) contenant du paraxylène, d'un premier raffinat intermédiaire (7) contenant de l'éthylbenzène, et d'un premier raffinat-2 (8) contenant de l'orthoxylène et du métaxylène,
- on effectue une séparation en lit mobile simulé de l'isomérat (11) issu de l'unité d'isomérisation (ISOM-1), dans l'unité de séparation (SMB-2) constituée d'au moins un adsorbeur contenant une pluralité de lits interconnectés et travaillant préférentiellement en boucle fermée, et ledit adsorbeur comprenant au moins cinq zones délimitées par les injections du flux (11) et du désorbant (17), et les soutirages d'un deuxième extrait (12) contenant du paraxylène, d'un deuxième raffinat intermédiaire (14) contenant de l'éthylbenzène, et d'un deuxième raffinat-2 (15) contenant de l'orthoxylène et du métaxylène,
- on distille le premier extrait (5) issu de l'unité de séparation (SMB-1) dans une colonne de distillation (EXT-1), pour récupérer une première fraction (6) enrichie en paraxylène,
- on mélange les deux raffinats-2, flux (8) et (15), pour former le flux (9) qui est distillé dans une colonne (RAF-2) de manière à éliminer sensiblement tout le désorbant, et pour soutirer une première fraction distillée (10),
- on alimente par le flux (10) une première unité d'isomérisation (ISOM-1) pour obtenir le premier isomérat (11),
- on mélange les deux raffinats intermédiaires, flux (7) et (14), pour former le flux (18) qui est distillé dans une colonne (RAFINTER) pour éliminer sensiblement tout le désorbant et pour soutirer une deuxième fraction distillée (19),
- on alimente par le flux (19) une deuxième unité d'isomérisation (ISOM-2) pour obtenir un deuxième isomérat (20) qui est recyclé en entrée de la colonne de distillation (S-1).

2. Procédé de production de paraxylène à haute pureté à partir d'une coupe xylènes contenant de l'éthylbenzène et des composés en C9+ selon la revendication 1, dans lequel l'unité d'isomérisation (ISOM1) fonctionne en phase liquide aux conditions suivantes :
- Température inférieure à 300°C, de préférence comprise entre 200 et 260°C,
- Pression inférieure à 4 MPa, de préférence comprise entre 2 et 3 MPa,
- Vitesse spatiale inférieure à 10 h⁻¹, de préférence comprise entre 2 h⁻¹ et 4 h⁻¹,
- Catalyseur comportant au moins une zéolithe présentant des canaux dont l'ouverture est définie par un anneau à 10 ou 12 atomes d'oxygène (10 MR ou 12 MR), préférentiellement un catalyseur comportant au moins une zéolithe présentant des canaux dont l'ouverture est définie par un anneau à 10 atomes d'oxygène (10 MR), et de manière encore plus préférée un catalyseur comportant une zéolithe de type ZSM-5.

3. Procédé de production de paraxylène à haute pureté à partir d'une coupe xylènes contenant de l'éthylbenzène et des composés en C9+, selon la revendication 1, dans lequel l'unité d'isomérisation (ISOM-2) fonctionne en phase gazeuse aux conditions suivantes :
- température supérieure à 300°C, de préférence de 360°C à 480°C,
- pression inférieure à 2,5 MPa et de préférence de 0,5 à 0,8 MPa,
- vitesse spatiale inférieure à 10 h⁻¹, de préférence comprise entre 0,5 h⁻¹ et 6 h⁻¹,
- rapport molaire hydrogène sur hydrocarbure inférieur à 10, et de préférence compris entre 3 et 6.

4. Procédé de production de paraxylène à haute pureté à partir d'une coupe xylènes contenant de l'éthylbenzène et des composés en C9+ selon la revendication 3, dans lequel le catalyseur utilisé pour l'unité d'isomérisation (ISOM-2) contient une zéolithe acide, par exemple de type structural MFI, MOR, MAZ, MTW, FAU et/ou EUO, et de manière préférée, contient une zéolithe de type structural EUO, et au moins un métal du groupe VIII de la classification périodique des éléments.

5. Procédé de production de paraxylène à haute pureté à partir d'une coupe xylènes contenant de l'éthylbenzène et des composés en C9+ selon la revendication 3, dans lequel le catalyseur utilisé pour l'unité d'isomérisation (ISOM-2) comporte au moins une zéolithe présentant des canaux dont l'ouverture est définie par un anneau à 10 ou 12 atomes d'oxygène (10 MR ou 12 MR), et au moins un métal du groupe VIII de teneur comprise entre 0,1 et 0,3% poids, bornes incluses.

6. Procédé de production de paraxylène à haute pureté à partir d'une coupe xylènes contenant de l'éthylbenzène et des composés en C9+ selon la revendication 1, dans lequel l'unité de séparation (SMB-1) utilise comme désorbant le PDEB.

7. Procédé de production de paraxylène à haute pureté à partir d'une coupe xylènes contenant de l'éthylbenzène et des composés en C9+ selon la revendication 1, dans lequel l'unité de séparation (SMB-2) utilise comme désorbant le toluène.

8. Procédé de production de paraxylène à haute pureté à partir d'une coupe xylènes contenant de l'éthylbenzène et des composés en C9+ selon la revendication 1, dans lequel les unités de séparation (SMB-1) et (SMB-2) contiennent de 6 à 24 lits, et de manière préférée de 8 à 15 lits répartis sur un ou plusieurs adsorbeurs, le nombre de lits étant ajusté de manière à ce que chaque lit ait de préférence une hauteur comprise entre 0,70 m et 1,40 m.

9. Procédé de production de paraxylène à haute pureté à partir d'une coupe xylènes contenant de l'éthylbenzène et des composés en C9+ selon la revendication 1, dans lequel la répartition de la quantité de solide adsorbant dans les unités de séparation (SMB-1) et (SMB-2) est la suivante :
• la quantité de solide adsorbant en zone 1 est de 16%±5%,
• la quantité de solide adsorbant en zone 2 est de 40%±5%,
• la quantité de solide adsorbant en zone 3A est de 16%±5%,
• la quantité de solide adsorbant en zone 3B est de 16%±5%,
• la quantité de solide adsorbant en zone 4 est de 12%±5%,
Les zones étant définies de la manière suivante :
- la zone 1 comprise entre l'injection du désorbant et le soutirage de l'extrait,
- La zone 2 comprise entre le soutirage de l'extrait et l'injection de la charge,
- La zone 3A comprise entre l'injection de la charge et le soutirage du raffinat intermédiaire,
- La zone 3B comprise entre le soutirage du raffinat intermédiaire et le soutirage du raffinat-2,
- La zone 4 comprise entre le soutirage du raffinat-2 et l'injection du désorbant.

10. Procédé de production de paraxylène à haute pureté à partir d'une coupe xylènes contenant de l'éthylbenzène et des composés en C9+ selon la revendication 1, dans lequel en ce qui concerne l'unité de séparation (SMB-1), le rapport volumétrique désorbant sur charge est d'au plus 1,7/1 et de manière préférée compris entre 1,5/1 et 0,4/1, bornes comprises.

11. Procédé de production de paraxylène à haute pureté à partir d'une coupe xylènes contenant de l'éthylbenzène et des composés en C9+ selon la revendication 1, dans lequel en ce qui concerne l'unité de séparation (SMB-1), le rapport des débits de raffinat intermédiaire et de raffinat 2 est compris entre 0,3/1 et 5/1, et de manière préférée compris entre 0,4/1 et 1/1 bornes comprises.

12. Procédé de production de paraxylène à haute pureté à partir d'une coupe xylènes contenant de l'éthylbenzène et des composés en C9+ selon la revendication 1, dans lequel en ce qui concerne l'unité de séparation (SMB-2), le rapport volumétrique désorbant sur charge est d'au plus 1,7/1 et de manière préférée compris entre 1,5/1 et 0,4/1 bornes comprises.

13. Procédé de production de paraxylène à haute pureté à partir d'une coupe xylènes contenant de l'éthylbenzène et des composés en C9+ selon la revendication 1, dans lequel en ce qui concerne l'unité de séparation (SMB-2), le rapport des débits de raffinat intermédiaire et de raffinat 2 est compris entre 0,3/1 et 5/1, et de manière préférée compris entre 0,4/1 et 1/1 bornes comprises.

14. Procédé de production de paraxylène à haute pureté à partir d'une coupe xylènes contenant de l'éthylbenzène et des composés en C9+ selon la revendication 1, procédé obtenu à partir du dégoulotage d'une unité de séparation existante, constituée de deux adsorbeurs en série, de la manière suivante :
- on connecte le dernier lit du premier adsorbeur au premier lit du premier adsorbeur via une ligne contenant au moins une pompe de recirculation, ce premier adsorbeur servant d'unité de séparation (SMB-1)
- on connecte le dernier lit du deuxième adsorbeur au premier lit du deuxième adsorbeur via une ligne contenant au moins une pompe de recirculation, ce deuxième adsorbeur servant d'unité de séparation (SMB-2).

15. Procédé de production de paraxylène à haute pureté à partir d'une coupe xylènes contenant de l'éthylbenzène et des composés en C9+ selon la revendication 1, dans lequel la configuration des deux unités de séparation (SMB-1) et (SMB-2) est à nombre de lits fixes dans chacune des zones chromatographiques de chacun des deux unités de séparation.

16. Procédé de production de paraxylène à haute pureté à partir d'une coupe xylènes contenant de l'éthylbenzène et des composés en C9+ selon la revendication 1, dans lequel :
- on distille le premier extrait (5) issu de l'unité de séparation (SMB-1) dans une colonne (EXT-1), pour récupérer une première fraction (6) enrichie en paraxylène,
- on distille le deuxième extrait (12) dans une colonne (EXT-2), pour récupérer une deuxième fraction (13) enrichie en paraxylène.

17. Procédé de production de paraxylène à haute pureté à partir d'une coupe xylènes contenant de l'éthylbenzène et des composés en C9+ selon la revendication 1, dans lequel les deux extraits (5) et (12) sont distillés dans une seule colonne d'extrait commune pour récupérer une seule fraction enrichie en paraxylène.

## Patentansprüche

1. Verfahren zum Herstellen von hochreinem Paraxylol aus einem Xylolschnitt, der Ethylbenzol und [C9+]-Verbindungen enthält, wobei das Verfahren zwei simulierte Wanderbett-Trenneinheiten (SMB-1 und SMB-2) und zwei Isomerisierungseinheiten (ISOM-1 und ISOM-2) verwendet, bestehend aus der folgenden Reihe von Schritten:
- Schicken des Einsatzmaterials (2) in eine Destillationssäule (S-1), aus der am Kopf ein Gemisch (3) abgezogen wird, das den Großteil des Metaxylols, Paraxylols, Ethylbenzols und mindestens einen Teil des Orthoxylols umfasst, und aus der am Boden ein Strom (4) aus Kohlenwasserstoffen mit 9-10 C-Atomen und der verbleibende Teil des Orthoxylols entnommen wird;
- Durchführen einer Trennung des Kopfgemischs (3) in der Trenneinheit (SMB-1), die mindestens einen Adsorber aufweist, der mehrere miteinander verbundene Betten enthält und in einem geschlossenen Kreislauf arbeitet, wobei der Adsorber mindestens fünf Zonen umfasst, die durch die Einspritzungen des Stroms (3) und des Desorbens (16) und den Entnahmen eines ersten Extrakts (5), der Paraxylol enthält, eines ersten Intermediärraffinats (7), das Ethylbenzol enthält, und eines ersten Raffinats-2 (8), das Orthoxylol und Metaxylol enthält, begrenzt sind,
- Durchführen einer Trennung im simulierten Wanderbett des Iomerats (11), dass aus der Isomerisierungseinheit (ISOM-1) stammt, in der Trenneinheit (SMB-2), die aus mindestens einem Adsorber besteht, der mehrere miteinander verbundene Betten enthält und vorzugsweise in einem geschlossenen Kreislauf arbeitet, und wobei der Adsorber mindestens fünf Zonen umfasst, die durch die Einspritzungen des Stroms (11) und des Desorbens (17) und den Entnahmen eines zweiten Extrakts (12), der Paraxylol enthält, eines zweiten Intermediärraffinats (14), das Ethylbenzol enthält, und eines zweiten Raffinats-2 (15), das Orthoxylol und Metaxylol enthält, begrenzt sind,
- Destillieren des ersten Extrakts (5), der aus der Trenneinheit (SMB-1) stammt, in einer Destillationskolonne (EXT-1), um eine erste Fraktion (6), die mit Paraxylol angereichert ist, zu gewinnen,
- Mischen der zwei Raffinate-2, Strom (8) und (15), um den Strom (9) zu bilden, der in einer Kolonne (RAF-2) destilliert wird, um im Wesentlichen das gesamte Desorbens zu entfernen, und um eine erste destillierte Fraktion (10) zu entnehmen,
- Einspeisen des Stroms (10) in eine erste Isomerisierungseinheit (ISOM-1), um das erste Isomerat (11) zu erhalten,
- Mischen der zwei Intermediärraffinate, Strom (7) und (14), um den Strom (18) zu bilden, der in einer Kolonne (RAFINTER) destilliert wird, um im Wesentlichen das gesamte Desorbens zu entfernen, und um eine zweite destillierte Fraktion (19) zu entnehmen,
- Einspeisen des Stroms (19) in eine zweite Isomerisierungseinheit (ISOM-2), um ein zweites Isomerat (20) zu erhalten, das an den Einlass der Destillationskolonne (S-1) zurückgeführt wird.

2. Verfahren zum Herstellen von hochreinem Paraxylol aus einem Xylolschnitt, der Ethylbenzol und [C9+]-Verbindungen enthält, nach Anspruch 1, wobei die Isomerisierungseinheit (ISOM1) in der Flüssigphase unter den folgenden Bedingungen arbeitet:
- Temperatur unterhalb von 300 °C, vorzugsweise im Bereich zwischen 200 und 260 °C,
- Druck unterhalb von 4 MPa, vorzugsweise im Bereich zwischen 2 und 3 MPa,
- Raumgeschwindigkeit von unter 10 h⁻¹, vorzugsweise im Bereich zwischen 2h⁻¹ und 4 h⁻¹,
- Katalysator, der mindestens einen Zeolithen mit Kanälen, deren Öffnung durch einen Ring mit 10 oder 12 Sauerstoffatomen definiert ist (10 MR oder 12 MR), vorzugsweise einen Katalysator mit mindestens einem Zeolithen mit Kanälen, deren Öffnung durch einen Ring mit 10 Sauerstoffatomen definiert ist (10 MR), und noch stärker bevorzugt einen Katalysator mit einem Zeolithen vom ZSM-5-Typ aufweist.

3. Verfahren zum Herstellen von hochreinem Paraxylol aus einem Xylolschnitt, der Ethylbenzol und [C9+]-Verbindungen enthält, nach Anspruch 1, wobei die Isomerisierungseinheit (ISOM-2) in der Gasphase unter den folgenden Bedingungen arbeitet:
- Temperatur oberhalb von 300°C, vorzugsweise von 360°C bis 480°C,
- Druck unterhalb von 2,5 MPa und vorzugsweise von 0,5 bis 0,8 MPa,
- Raumgeschwindigkeit von unter 10 h⁻¹, vorzugsweise im Bereich zwischen 0,5h⁻¹ und 6 h⁻¹,
- Molverhältnis von Wasserstoff zu Kohlenwasserstoff kleiner 10 und bevorzugt im Bereich zwischen 3 und 6.

4. Verfahren zum Herstellen von hochreinem Paraxylol aus einem Xylolschnitt, der Ethylbenzol und [C9+]-Verbindungen enthält, nach Anspruch 3, wobei der Katalysator, der für die Isomerisierungseinheit (ISOM-2) verwendet wird, einen sauren Zeolithen, zum Beispiel vom Strukturtyps MFI, MOR, MAZ, MTW, FAU und/oder EUO, und vorzugsweise einen Zeolithen vom Strukturtyp EUO enthält, und mindestens ein Metall der Gruppe VIII des Periodensystems der Elemente enthält.

5. Verfahren zum Herstellen von hochreinem Paraxylol aus einem Xylolschnitt, der Ethylbenzol und [C9+]-Verbindungen enthält, nach Anspruch 3, wobei der für die Isomerisierungseinheit (ISOM-2) verwendete Katalysator mindestens einen Zeolithen mit Kanälen, deren Öffnung durch einen Ring mit 10 oder 12 Sauerstoffatomen (10 MR oder 12 MR) definiert ist, und mindestens ein Metall der Gruppe VIII in einem Anteil im Bereich zwischen 0,1 und 0,3 Gew.-%, einschließlich der Grenzwerte, aufweist.

6. Verfahren zum Herstellen von hochreinem Paraxylol aus einem Xylolschnitt, der Ethylbenzol und [C9+]-Verbindungen enthält, nach Anspruch 1, wobei die Trenneinheit (SMB-1) als Desorbens PDEB verwendet.

7. Verfahren zum Herstellen von hochreinem Paraxylol aus einem Xylolschnitt, der Ethylbenzol und [C9+]-Verbindungen enthält, nach Anspruch 1, wobei die Trenneinheit (SMB-2) als Desorbens Toluol verwendet.

8. Verfahren zum Herstellen von hochreinem Paraxylol aus einem Xylolschnitt, der Ethylbenzol und [C9+]-Verbindungen enthält, nach Anspruch 1, wobei die Trenneinheiten (SMB-1) und (SMB-2) 6 bis 24 Betten und vorzugsweise 8 bis 15 Betten enthalten, die auf einem oder mehreren Adsorbern verteilt sind, wobei die Anzahl der Betten so angepasst ist, dass jedes Bett vorzugsweise eine Höhe im Bereich zwischen 0,70 m und 1,40 m hat.

9. Verfahren zum Herstellen von hochreinem Paraxylol aus einem Xylolschnitt, der Ethylbenzol und [C9+]-Verbindungen enthält, nach Anspruch 1, wobei die Verteilung der Menge an Feststoffadsorbens in den Trenneinheiten (SMB-1) und (SMB-2) wie folgt ist:
• die Menge an Feststoffadsorbens in der Zone 1 beträgt 16 % ± 5 %,
• die Menge an Feststoffadsorbens in der Zone 2 beträgt 40 % ± 5 %,
• die Menge an Feststoffadsorbens in der Zone 3A beträgt 16 % ± 5 %,
• die Menge an Feststoffadsorbens in der Zone 3B beträgt 16 % ± 5 %,
• die Menge an Feststoffadsorbens in der Zone 4 beträgt 12 % ± 5 %,
wobei die Zonen wie folgt definiert sind:
- die Zone 1 liegt im Bereich zwischen der Desorbenseinspritzung und der Extraktentnahme,
- die Zone 2 liegt im Bereich zwischen der Extraktentnahme und der Einsatzmaterialeinspritzung,
- die Zone 3A liegt im Bereich zwischen der Einsatzmaterialeinspritzung und der Intermediärraffinatentnahme,
- die Zone 3B liegt im Bereich zwischen der Intermediärraffinatentnahme und der Raffinat- 2- Entnahme,
- die Zone 4 liegt im Bereich zwischen der Raffinat-2-entnahme und der Desorbenseinspritzung.

10. Verfahren zum Herstellen von hochreinem Paraxylol aus einem Xylolschnitt, der Ethylbenzol und [C9+]-Verbindungen enthält, nach Anspruch 1, wobei in Bezug auf die Trenneinheit (SMB-1) das volumetrische Verhältnis von Desorbens zu Einsatzmaterial bei höchstens 1,7/1 und vorzugsweise im Bereich zwischen 1,5/1 und 0,4/1, einschließlich der Grenzwerte, liegt.

11. Verfahren zum Herstellen von hochreinem Paraxylol aus einem Xylolschnitt, der Ethylbenzol und [C9+]-Verbindungen enthält, nach Anspruch 1, wobei in Bezug auf die Trenneinheit (SMB-1) das Verhältnis der Flussmengen von Intermediärraffinat und Raffinat 2 im Bereich zwischen 0,3/1 und 5/1, und vorzugsweise im Bereich zwischen 0,4/1 und 1/1, einschließlich der Grenzwerte, liegt.

12. Verfahren zum Herstellen von hochreinem Paraxylol aus einem Xylolschnitt, der Ethylbenzol und [C9+]-Verbindungen enthält, nach Anspruch 1, wobei in Bezug auf die Trenneinheit (SMB-2) das volumetrische Verhältnis von Desorbens zu Einsatzmaterial bei höchstens 1,7/1 und vorzugsweise im Bereich zwischen 1,5/1 und 0,4/1, einschließlich der Grenzwerte, liegt.

13. Verfahren zum Herstellen von hochreinem Paraxylol aus einem Xylolschnitt, der Ethylbenzol und [C9+]-Verbindungen enthält, nach Anspruch 1, wobei in Bezug auf die Trenneinheit (SMB-2) das Verhältnis der Flussmengen von Intermediärraffinat und Raffinat 2 im Bereich zwischen 0,3/1 und 5/1, und vorzugsweise im Bereich zwischen 0,4/1 und 1/1, einschließlich der Grenzwerte, liegt.

14. Verfahren zum Herstellen von hochreinem Paraxylol aus einem Xylolschnitt, der Ethylbenzol und [C9+]-Verbindungen enthält, nach Anspruch 1, wobei das Verfahren ausgehend von der Erweiterung einer vorhandenen Trenneinheit, die aus zwei Adsorbern in Reihe besteht, wie folgt erhalten wird:
- Verbinden des letzten Betts des ersten Adsorbers mit dem ersten Bett des ersten Absorbers über eine Leitung, die mindestens eine Umlaufpumpe enthält, wobei dieser erste Adsorber als Trenneinheit (SMB-1) dient
- Verbinden des letzten Betts des zweiten Adsorbers mit dem ersten Bett des zweiten Absorbers über eine Leitung, die mindestens eine Umlaufpumpe enthält, wobei dieser zweite Adsorber als Trenneinheit (SMB-2) dient.

15. Verfahren zum Herstellen von hochreinem Paraxylol aus einem Xylolschnitt, der Ethylbenzol und [C9+]-Verbindungen enthält, nach Anspruch 1, wobei die Konfiguration der beiden Trenneinheiten (SMB-1) und (SMB-2) eine feste Anzahl an Betten in jeder der Chromatographiezonen jeder der beiden Trenneinheiten aufweist.

16. Verfahren zum Herstellen von hochreinem Paraxylol aus einem Xylolschnitt, der Ethylbenzol und [C9+]-Verbindungen enthält, nach Anspruch 1, wobei:
- Destillieren des ersten Extrakts (5), der aus der Trenneinheit (SMB-1) stammt, in einer Kolonne (EXT-1), um eine erste Fraktion (6), die mit Paraxylol angereichert ist, zu gewinnen,
- Destillieren des zweiten Extrakts (12) in einer Kolonne (EXT-2), um eine zweite Fraktion (13), die mit Paraxylol angereichert ist, zu gewinnen.

17. Verfahren zum Herstellen von hochreinem Paraxylol aus einem Xylolschnitt, der Ethylbenzol und [C9+]-Verbindungen enthält, nach Anspruch 1, wobei die beiden Extrakte (5) und (12) in einer einzigen gemeinsamen Extraktionskolonne destilliert werden, um eine einzige Fraktion, die mit Paraxylol angereichert ist, zu gewinnen.

## Claims

1. Process for the production of high-purity paraxylene from a xylenes cut containing ethylbenzene and C9+ compounds, a process using two simulated moving bed separation units (SMB-1 et SMB-2), and two isomerization units (ISOM-1 et ISOM-2) consisting of the following series of stages:
- the feedstock (2) is sent to a distillation column (S-1) from where a mixture (3) is drawn off at the top comprising the major part of the metaxylene, paraxylene, ethylbenzene, and at least a part of the orthoxylene, and from where a flow (4) of C9-C10 hydrocarbons and the remaining part of the orthoxylene is drawn off at the bottom.
- the mixture from the top (3) is separated in the separation unit (SMB-1) comprising at least one adsorber containing a plurality of interconnected beds and operating in a closed loop, said adsorber comprising at least five zones delimited by the injections of the flow (3) and of the desorbent (16), and a first extract (5) containing paraxylene, a first intermediate raffinate (7) containing ethylbenzene, and a first raffinate-2 (8) containing orthoxylene and metaxylene are drawn off,
- a simulated moving bed separation of the isomerate (11) originating from the isomerization unit (ISOM-1), is carried out in the separation unit (SMB-2) constituted by at least one adsorber containing a plurality of interconnected beds and operating preferentially in a closed loop, and said adsorber comprising at least five zones delimited by the injections of the flow (11) and of the desorbent (17), and a second extract (12) containing paraxylene, a second intermediate raffinate (14) containing ethylbenzene, and a second raffinate-2 (15) containing orthoxylene and metaxylene are drawn off,
- the first extract (5) originating from the separation unit (SMB-1) is distilled in a distillation column (EXT-1), in order to recover a first fraction (6) enriched with paraxylene,
- the two raffinates-2, flows (8) and (15), are mixed in order to form the flow (9) which is distilled in a column (RAF-2) so as to eliminate substantially all the desorbent, and in order to draw off a first distilled fraction (10),
- a first isomerization unit (ISOM-1) is supplied with the flow (10), in order to obtain the first isomerate (11),
- the two intermediate raffinates, flows (7) and (14), are mixed in order to form the flow (18) which is distilled in a column (RAFINTER) in order to substantially eliminate all of the desorbent and in order to draw off a second distilled fraction (19),
- a second isomerization unit (ISOM-2) is supplied with the flow (19), in order to obtain a second isomerate (20), which is recycled to the inlet of the separation column (S-1).

2. Process for the production of high-purity paraxylene from a xylenes cut containing ethylbenzene and C9+ compounds according to claim 1, in which the isomerization unit (ISOM-1) operates in liquid phase under the following conditions:
- Temperature less than 300°C, preferably comprised between 200 and 260°C,
- Pressure less than 4MPa, preferably comprised between 2 and 3 MPa,
- Hourly space velocity less than 10 h⁻¹, preferably comprised between 2h⁻¹ and 4 h⁻¹,
- Catalyst comprising at least one zeolite having channels the opening of which is defined by a ring with 10 or 12 oxygen atoms (10 MR or 12 MR), preferentially a catalyst comprising at least one zeolite having channels the opening of which is defined by a ring with 10 oxygen atoms (10 MR), and even more preferably, a catalyst including a zeolite of the ZSM-5 type.

3. Process for the production of high-purity paraxylene from a xylenes cut containing ethylbenzene and C9+ compounds according to claim 1, in which the isomerization unit (ISOM-2) operates in liquid phase under the following conditions:
- temperature greater than 300 °C, preferably from 360 °C to 480 °C,
- pressure less than 2.5 MPa and preferably from 0.5 to 0.8 MPa,
- hourly space velocity less than 10 h⁻¹, preferably comprised between 0.5h⁻¹ and 6 h⁻¹,
- hydrogen to hydrocarbon molar ratio less than 10, and preferably comprised between 3 and 6.

4. Process for the production of high-purity paraxylene from a xylenes cut containing ethylbenzene and C9+ compounds according to claim 3, in which the catalyst used for the isomerization unit (ISOM-2) contains an acid zeolite, for example of the MFI, MOR, MAZ, MTW, FAU and/or EUO structure type, and preferably, contains a zeolite of the EUO structure type, and at least one metal of group VIII of the periodic table.

5. Process for the production of high-purity paraxylene from a xylenes cut containing ethylbenzene and C9+ compounds according to claim 3, in which the catalyst used for the isomerization unit (ISOM-2) contains at least one zeolite having channels the opening of which is defined by a ring with 10 to 12 oxygen atoms (10 MR or 12 MR), and at least one group VIII metal at a content comprised between 0.1 and 0.3% by weight, inclusive.

6. Process for the production of high-purity paraxylene from a xylenes cut containing ethylbenzene and C9+ compounds according to claim 1, in which the separation unit (SMB-1) uses PDEB as desorbent.

7. Process for the production of high-purity paraxylene from a xylenes cut containing ethylbenzene and C9+ compounds according to claim 1, in which the separation unit (SMB-2) uses toluene as desorbent.

8. Process for the production of high-purity paraxylene from a xylenes cut containing ethylbenzene and C9+ compounds according to claim 1, in which the separation units (SMB1) and (SMB-2) contain from 6 to 24 beds, and preferably from 8 to 15 beds, distributed over one or more adsorbers, the number of beds being adjusted so that each bed preferably has a height comprised between 0.70 m and 1.40 m.

9. Process for the production of high-purity paraxylene from a xylenes cut containing ethylbenzene and C9+ compounds according to claim 1, in which the distribution of the quantity of solid adsorbent in the separation units (SMB-1) and (SMB-2) is as follows:
• the quantity of solid adsorbent in zone 1 is 16%±5%,
• the quantity of solid adsorbent in zone 2 is 40%±5%,
• the quantity of solid adsorbent in zone 3A is 16%±5%,
• the quantity of solid adsorbent in zone 3B is 16%±5%,
• the quantity of solid adsorbent in zone 4 is 12%±5%,
The zones being defined as follows:
- zone 1 comprised between the injection of the desorbent and the draw off of the extract,
- zone 2 comprised between the draw-off of the extract and the injection of the feedstock,
- zone 3A comprised between the injection of the feedstock and the draw-off of the intermediate raffinate,
- zone 3B comprised between the draw off of the intermediate raffinate and the draw-off of the raffinate-2,
- zone 4 comprised between the draw-off of the raffinate-2 and the injection of the desorbent.

10. Process for the production of high-purity paraxylene from a xylenes cut containing ethylbenzene and C9+ compounds according to claim 1, in which, with respect to the separation unit (SMB-1), the ratio by volume of desorbent to feedstock is at least 1.7/1 and preferably comprised between 1.5/1 and 0.4/1, inclusive.

11. Process for the production of high-purity paraxylene from a xylenes cut containing ethylbenzene and C9+ compounds according to claim 1, in which, with respect to the separation unit (SMB-1), the ratio of the flow rates of intermediate raffinate and of raffinate-2 is comprised between 0.3/1 and 5/1, and preferably comprised between 0.4/1 and 1/1, inclusive.

12. Process for the production of high-purity paraxylene from a xylenes cut containing ethylbenzene and C9+ compounds according to claim 1, in which, with respect to the separation unit (SMB-2), the ratio by volume of desorbent to feedstock is at least 1.7/1 and preferably comprised between 1.5/1 and 0.4/1, inclusive.

13. Process for the production of high-purity paraxylene from a xylenes cut containing ethylbenzene and C9+ compounds according to claim 1, in which, with respect to the separation unit (SMB-2), the ratio of the flow rates of intermediate raffinate and of raffinate-2 is comprised between 0.3/1 and 5/1, and preferably comprised between 0.4/1 and 1/1, inclusive.

14. Process for the production of high-purity paraxylene from a xylenes cut containing ethylbenzene and C9+ compounds according to claim 1, a process obtained based on the debottlenecking of an existing separation unit, constituted by two adsorbers in series, as follows:
- the last bed of the first adsorber is connected to the first bed of the first adsorber via a line containing at least one recirculation pump, this first adsorber acting as a separation unit (SMB-1)
- the last bed of the second adsorber is connected to the first bed of the second adsorber via a line containing at least one recirculation pump, this second adsorber acting as a separation unit (SMB-2)

15. Process for the production of high-purity paraxylene from a xylenes cut containing ethylbenzene and C9+ compounds according to claim 1, in which the configuration of the two separation units (SMB-1) and (SMB-2) has a fixed number of beds in each of the chromatographic zones of each of the two separation units.

16. Process for the production of high-purity paraxylene from a xylenes cut containing ethylbenzene and C9+ compounds according to claim 1, in which:
- the first extract (5) originating from the separation unit (SMB-1) is distilled in a column (EXT-1), in order to recover a first fraction (6) enriched with paraxylene,
- the second extract (12) is distilled in a column (EXT-2), in order to recover a second fraction (13) enriched with paraxylene.

17. Process for the production of high-purity paraxylene from a xylenes cut containing ethylbenzene and C9+ compounds according to claim 1, in which the two extracts (5) and (12) are distilled in a single common extraction column in order to recover a single fraction enriched with paraxylene.
